(19) Europäisches Patentamt
European Patent Office
Office européen des brevets

(11) EP 2 229 815 B1

(12) **EUROPÄISCHE PATENTSCHRIFT**

(45) Veröffentlichungstag und Bekanntmachung des Hinweises auf die Patenterteilung:
**29.05.2013 Patentblatt 2013/22**

(51) Int Cl.:
***A01N 43/56*** *(2006.01)* ***A01N 43/653*** *(2006.01)*
***A01P 3/00*** *(2006.01)*

(21) Anmeldenummer: **10163282.6**

(22) Anmeldetag: **28.09.2004**

(54) **Synergistische fungizide Wirkstoffkombinationen**

Synergistic combinations of fungicidal compounds

Combinaisons de composés fongicides synergétiques

(84) Benannte Vertragsstaaten:
**AT BE BG CH CY CZ DE DK EE ES FI FR GB GR HU IE IT LI LU MC NL PL PT RO SE SI SK TR**

(43) Veröffentlichungstag der Anmeldung:
**22.09.2010 Patentblatt 2010/38**

(62) Dokumentnummer(n) der früheren Anmeldung(en) nach Art. 76 EPÜ:
**04765648.3 / 1 675 461**

(73) Patentinhaber: **Bayer CropScience AG**
**40789 Monheim (DE)**

(72) Erfinder:
- **Wachendorff-Neumann, Ulrike, Dr.**
  **56566 Neuwied (DE)**
- **Dahmen, Peter, Dr.**
  **41470 Neuss (DE)**
- **Dunkel, Ralf, Dr.**
  **42799 Leichlingen (DE)**
- **Elbe, Hans-Ludwig, Dr.**
  **42329 Wuppertal (DE)**
- **Suty-Heinze, Anne**
  **40764 Langenfeld (DE)**
- **Rieck, Heiko, Dr.**
  **51399 Burscheid (DE)**

(56) Entgegenhaltungen:
**WO-A-99/31979 WO-A1-03/070705**

Bemerkungen:
Die Akte enthält technische Angaben, die nach dem Eingang der Anmeldung eingereicht wurden und die nicht in dieser Patentschrift enthalten sind.

EP 2 229 815 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft neue Wirkstoffkombinationen, die aus Bixafen einerseits und bekannten Triazolen andererseits bestehen und sehr gut zur Bekämpfung von unerwünschten phytopathogenen Pilzen geeignet sind.

Es ist bereits bekannt, dass bestimmte Carboxamide fungizide Eigenschaften besitzen. So sind z.B. *N*-(3',4'-Dichlor-5-fluor-1,1'-biphenyl-2-yl)-3-(difluormethyl)-1-methyl-1*H*-pyrazol-4-carboxamid aus DE-A 102 15 292, 3-(Trifluormethyl)-*N*-{3'-fluor-4'-[(E)-(methoxyimino)methyl]-1,1'-biphenyl-2-yl}-1-methyl-1*H*-pyrazol-4-carboxamid aus WO 02/08197, sowie *N*-(3',4'-Dichlor-1,1'-biphenyl-2-yl)-5-fluor-1,3-dimethyl-1*H*-pyrazol-4-carboxamid aus WO 00/14071 bekannt. Die Wirksamkeit dieser Stoffe ist gut, lässt aber bei niedrigen Aufwandmengen in manchen Fällen zu wünschen übrig. Ferner ist schon bekannt, dass zahlreiche Triazol-Derivate, Anilin-Derivate, Dicarboximide und andere Heterocyclen zur Bekämpfung von Pilzen eingesetzt werden können (vgl. EP-A 0 040 345, DE-A 22 01 063, DE-A 23 24 010, Pesticide Manual, 9th. Edition (1991), Seiten 249 und 827, EP-A 0 382 375 und EP-A 0 515 901). Auch die Wirkung dieser Stoffe ist aber bei niedrigen Aufwandmengen nicht immer ausreichend. Ferner ist bereits bekannt, dass 1-(3,5-Dimethyl-isoxazol-4-sulfonyl)-2-chlor-6,6-difluor-[1,31-dioxolo-[4,5f]-benzimidazol fungizide Eigenschaften besitzt (vgl. WO 97/06171). Außerdem ist auch bekannt, dass substituierte Halogenpyrimidine fungizide Eigenschaften besitzen (vgl. DE-A1-196 46 407, EP-B-712 396). Ferner sind fungizide Mischungen auf der Basis einer Amidverbindung der Formel I (A-CO-$NR^1R^2$) und einem Azolderivat bekannt (WO 99/31979). Die Definition der generischen Formel **I** (A-CO-$NR^1R^2$) in der WO 99/31979 umfasst eine sehr große Zahl an möglichen Verbindungen. Nur in einer von vielen möglichen Definitionen ist A ein Pyrazolylrest. Die Definition von $R^2$ als Biphenylrest ist ebenfalls nur eine von sehr vielen in der D1 beschriebenen Möglichkeiten. Der Unterschied gegenüber der D1 besteht nicht nur in den starken strukturellen Abweichungen des erfindungsgemäßen Kombinationspartners (1-1) gegenüber den in der D1 bevorzugten Amidverbindungen sondern auch in Abweichungen bei der Auswahl der Triazol-Kombinationspartner. Die in der vorliegenden Erfindung beanspruchten Triazol-Kombinationspartner (3-15) Prothioconazole und (3-21) Bitertanol sind in der D1 nicht offenbart. Schließlich offenbart die WO 03/070705 A1 Carboxamide der Formel I, unter anderem das Carboxamid *N*-(3', 4'-Dichlor-5-fluor-1,1'-biphenyl-2-yl)-3-(difluormethyl)-1-methyl-1*H*-pyrazol-4-carboxamid (Bixafen). Die Möglichkeit der Mischung der Carboxamide mit weiteren bekannten Fungiziden wird erwähnt, spezifische Kombinationen von Bixafen und Azolen sind aber nicht offenbart.

Es wurden nun neue Wirkstoffkombinationen mit sehr guten fungiziden Eigenschaften gefunden, enthaltend das Carboxamid (1-1) *N*-(3',4'-Dichlor-5-fluor-1,1'-biphenyl-2-yl)-3-(difluormethyl)-1-methyl-1*H*-pyrazol-4-carboxamid (bekannt aus WO 03/070705) und mindestens einen Wirkstoff, der aus der Gruppe (3) ausgewählt ist:

(3-3) Propiconazole (bekannt aus DE-A 25 51 560) der Formel

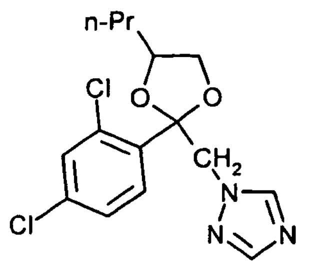

(3-6) Cyproconazole (bekannt aus DE-A 34 06 993) der Formel

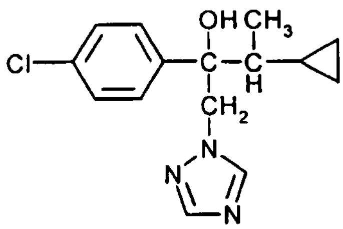

(3-12) Epoxiconazole (bekannt aus EP-A 0 196 038) der Formel

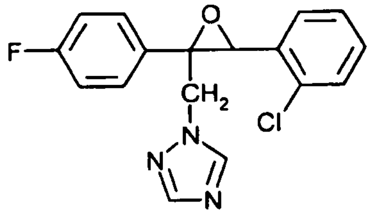

(3-15) Prothioconazole (bekannt aus WO 96/16048) der Formel

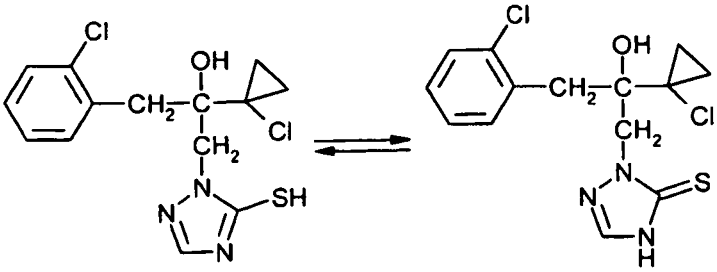

(3-17) Tebuconazole (bekannt aus EP-A 0 040 345) der Formel

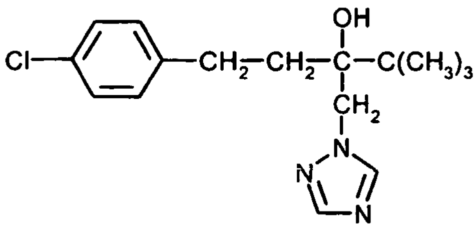

(3-19) Metconazole (bekannt aus EP-A 0 329 397) der Formel

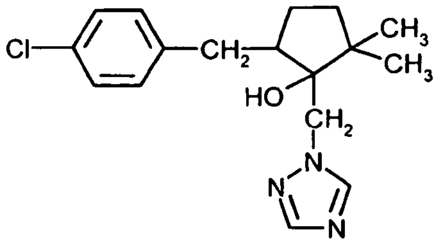

und

(3-21) Bitertanol (bekannt aus DE-A 23 24 010) der Formel

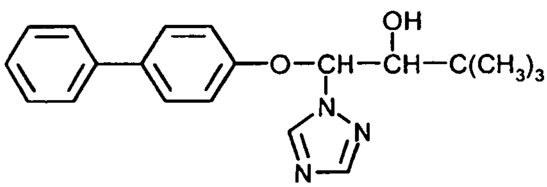

.

Die Wirkstoffkombinationen B enthalten neben dem Carboxamid (1-1) *N*-(3',4'-Dichlor-5-fluor-1,1'-biphenyl-2-yl)-3-(difluormethyl)-1-methyl-1*H*-pyrazol-4-carboxamid auch ein Triazol worin das Triazol aus der folgenden Liste ausgewählt ist:

(3-3) Propiconazole

(3-6) Cyproconazole

(3-12) Epoxiconazole

(3-15) Prothioconazole

(3-17) Tebuconazole

(3-19) Metconazole und

(3-21) Bitertanol.

Hervorgehoben sind die in der folgenden Tabelle 2 angeführten Wirkstoffkombinationen B:

**Tabelle 2: Wirkstoffkombinationen B**

| Nr. | Carboxamid der Formel (I) | Azol der Formel (III) |
|---|---|---|
| B-1 | (1-1) N-(3',4'-Dichlor-5-fluor-1,1'-biphenyl-2-yl)-3-(difluormethyl)-1-methyl-1H-pyrazol-4-carboxamid | (3-3) Propiconazole |
| B-2 | (1-1) N-(3',4'-Dichlor-5-fluor-1,1'-biphenyl-2-yl)-3-(difluormethyl)-1-methyl-1H-pyrazol-4-carboxamid | (3-6) Cyproconazole |
| B-3 | (1-1) N-(3',4'-Dichlor-5-fluor-1,1'-biphenyl-2-yl)-3-(difluormethyl)-1-methyl-1H-pyrazol-4-carboxamid | (3-15) Prothioconazole |
| B-4 | (1-1) N-(3',4'-Dichlor-5-fluor-1,1'-biphenyl-2-yl)-3-(difluormethyl)-1-methyl-1H-pyrazol-4-carboxamid | (3-17) Tebuconazole |
| B-5 | (1-1) N-(3',4'-Dichlor-5-fluor-1,1'-biphenyl-2-yl)-3-(difluormethyl)-1-methyl-1H-pyrazol-4-carboxamid | (3-21) Bitertanol |
| B-43 | (1-1) N-(3',4'-Dichlor-5-fluor-1,1'-biphenyl-2-yl)-3-(difluormethyl)- 1-methyl-H-pyrazol-4-carboxamid | (3-19) Metconazole |

Die erfindungsgemäßen Wirkstoffkombinationen können darüber hinaus auch weitere fungizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im Allgemeinen enthalten die erfindungsgemäßen Kombinationen den Wirkstoff N-(3',4'-Dichlor-5-fluor-1,1'-biphenyl-2-yl)-3-(difluormethyl)-1-methyl-1H-pyrazol-4-carboxamid und einen Mischpartner aus der Gruppe (3) in den in der nachfolgenden Tabelle 21 beispielhaft angegebenen Mischungsverhältnissen.

Die Mischungsverhältnisse basieren auf Gewichtsverhältnissen. Das Verhältnis ist zu verstehen als Wirkstoff der Formel (I) : Mischpartner

**Tabelle 21: Mischungsverhältnisse**

| Mischpartner | | bevorzugtes Mischungsverhältnis | besonders bevorzugtes Mischungsverhältnis |
|---|---|---|---|
| Gruppe (3): | Triazole ohne (3-15) | 50 : 1 bis 1 : 50 | 20 : 1 bis 1 : 20 |
| (3-15): | Prothioconazole | 50 : 1 bis 1 : 50 | 10 : 1 bis 1 : 20 |

Das Mischungsverhältnis ist in jedem Fall so zu wählen, dass eine synergistische Mischung erhalten wird. Die Mischungsverhältnisse zwischen dem Carboxamid (1-1) N-(3',4'-Dichlor-5-fluor-1,1'-biphenyl-2-yl)-3-(difluormethyl)-1-methyl-1H-pyrazol-4-carboxamid (Bixafen) und einem Mischpartner aus der Gruppe (3) können auch zwischen den einzelnen Verbindungen einer Gruppe variieren.

Die erfindungsgemäßen Wirkstoffkombinationen besitzen sehr gute fungizide Eigenschaften und lassen sich zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Wirkstoffkombinationen eignen sich besonders gut zur Bekämpfung von Erysiphe graminis, Pyrenophora teres und Leptosphaeria nodorum.

Beispielhaft, aber nicht begrenzend, seien einige Erreger von pilzlichen Erkrankungen, die unter die oben aufgezählten Oberbegriffe fallen, genannt:

Pythium-Arten, wie z.B. *Pythium ultimum*; Phytophthora-Arten, wie z.B. *Phytophthora infestans;* Pseudoperonospora-Arten, wie z.B. *Pseudoperonospora humuli* oder *Pseudoperonospora cubensis;* Plasmopara-Arten, wie z.B. *Plasmopara viticola;* Bremia-Arten, wie z.B. *Bremia lactucae;* Peronospora-Arten, wie z.B. *Peronospora pisi* oder *P. brassicae;* Erysiphe-Arten, wie z.B. *Erysiphe graminis;* Sphaerotheca-Arten, wie z.B. *Sphaerotheca fuliginea;* Podosphaera-Arten, wie z.B. *Podosphaera leucotricha;* Venturia-Arten, wie z.B. *Venturia inaequalis;* Pyrenophora-Arten, wie z.B. *Pyrenophora teres* oder *P. graminea* (Konidienform: Drechslera, Syn: Helminthosporium); Cochliobolus-Arten, wie z.B. *Cochliobolus sativus* (Konidienform: Drechslera, Syn: Helminthosporium); Uromyces-Arten, wie z.B. *Uromyces appendiculatus;* Puccinia-Arten, wie z.B. *Puccinia recondita;* Sclerotinia-Arten, wie z.B. *Sclerotinia sclerotiorum;* Tilletia-Arten, wie z.B. *Tilletia caries;* Ustilago-Arten, wie z.B. *Ustilago nuda* oder *Ustilago avenae;* Pellicularia-Arten, wie z.B. *Pellicularia sasakii*; Pyricularia-Arten, wie z.B. *Pyricularia oryzae;* Fusarium-Arten, wie z.B. *Fusarium culmorum;* Botrytis-Arten, wie z.B. *Botrytis cinerea*; Septoria-Arten, wie z.B. *Septoria nodorum;* Leptosphaeria-Arten, wie z.B. *Leptosphaeria nodorum;* Cercospora-Arten, wie z.B. *Cercospora canescens;* Alternaria-Arten, wie z.B. *Alternaria brassicae;* Pseudocercosporella-Arten, wie z.B. *Pseudocercosporella herpotrichoides,* Rhizoctonia-Arten, wie z.B. *Rhizoctonia solani.*

Die gute Pflanzenverträglichkeit der Wirkstoffkombinationen in den zur Bekämpfung von Pflanzenkrankheiten notwendigen Konzentrationen erlaubt eine Behandlung der gesamten Pflanzen (oberirdische Pflanzenteile und Wurzeln), von Pflanz- und Saatgut, und des Bodens. Die erfindungsgemäßen Wirkstoffkombinationen können zur Blattapplikation oder auch als Beizmittel eingesetzt werden.

Die erfindungsgemäßen Wirkstoffkombinationen eignen sich auch zur Steigerung des Ernteertrages. Sie sind außerdem mindertoxisch und weisen eine gute Pflanzenverträglichkeit auf.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfingdungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit

der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (zB. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäßen Wirkstoffkombinationen können in Abhängigkeit von ihren jeweiligen physikalischen und/oder chemischen Eigenschaften in die üblichen Formulierungen überführt werden, wie Lösungen, Emulsionen, Suspensionen, Pulver, Stäubemittel, Schäume, Pasten, lösliche Pulver, Granulate, Aerosole, Suspensions-Emulsions-Konzentrate, Wirkstoff-imprägnierte Natur - und synthetische Stoffe sowie Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiernnitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier- und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, kömige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Der Wirkstoffgehalt der aus den handelsüblichen Formulierungen bereiteten Anwendungsformen kann in weiten Bereichen variieren. Die Wirkstoffkonzentration der Anwendungsformen zum Bekämpfen tierischer Schädlingen wie Insekten und Akariden kann von 0,0000001 bis zu 95 Gew.-% Wirkstoff, vorzugsweise zwischen 0,0001 und 1 Gew.-% liegen. Die Anwendung geschieht in einer den Anwendungsformen angepassten üblichen Weise.

Die Formulierungen zur Bekämpfung unerwünschter phytopathogener Pilze enthalten im Allgemeinen zwischen 0,1 und 95 Gew.-% Wirkstoffe, vorzugsweise zwischen 0,5 und 90 %.

Die erfindungsgemäßen Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver, Stäubemittel und Granulate, angewendet werden. Die Anwendung geschieht in üblicher Weise, z.B. durch Gießen (drenchen), Tröpfchenbewässerung" Verspritzen, Versprühen, Verstreuen, Verstäuben, Verschäumen, Bestreichen, Verstreichen, Trockenbeizen, Feuchtbeizen, Nassbeizen, Schlämmbeizen, Inkrustieren usw.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen.

Beim Einsatz der erfindungsgemäßen Wirkstoffkombinationen können die Aufwandmengen je nach Applikationsart innerhalb eines größeren Bereichs variiert werden. Bei der Behandlung von Pflanzenteilen liegen die Aufwandmengen an Wirkstoffkombination im allgemeinen zwischen 0,1 und 10 000 g/ha, vorzugsweise zwischen 10 und 1 000 g/ha. Bei der Saatgutbehandlung liegen die Aufwandmengen an Wirkstoffkombination im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoffkombination im allgemeinen zwischen 0,1 und 10 000 g/ha, vorzugsweise zwischen 1 und 5 000 g/ha.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die gute fungizide Wirkung der erfindungsgemäßen Wirkstoffkombinationen geht aus den nachfolgenden Beispielen hervor. Während die einzelnen Wirkstoffe in der fungiziden Wirkung Schwächen aufweisen, zeigen die Kombinationen eine Wirkung, die über eine einfache Wirkungssummierung hinausgeht.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

Die zu erwartende fungizide Wirkung für eine gegebene Kombination zweier Wirkstoffe kann nach S.R. Colby ("Calculating Synergistic and Antagonistic Responses of Herbicide Combinations", Weeds 1967, 15, 20-22) wie folgt berechnet werden:

Wenn

X den *Wirkungsgrad* beim Einsatz des Wirkstoffes A in einer Aufwandmenge von *m g/ha* bedeutet,

Y den *Wirkungsgrad* beim Einsatz des Wirkstoffes B in einer Aufwandmenge von *n g/ha* bedeutet und

E den *Wirkungsgrad* beim Einsatz der Wirkstoffe A und B in Aufwandmengen von *m und* n *g/ha* bedeutet, dann ist

$$E = X + Y - \frac{X \times Y}{100}$$

Dabei wird der Wirkungsgrad in % ermittelt. Es bedeutet 0 % ein Wirkungsgrad, der demjenigen der Kontrolle entspricht, während ein Wirkungsgrad von 100 % bedeutet, dass kein Befall beobachtet wird.

Ist die tatsächliche fungizide Wirkung größer als berechnet, so ist die Kombination in ihrer Wirkung überadditiv, d.h. es liegt ein synergistischer Effekt vor. In diesem Fall muss der tatsächlich beobachtete Wirkungsgrad größer sein als der aus der oben angeführten Formel errechnete Wert für den erwarteten Wirkungsgrad (E).

Die Erfindung wird durch die folgenden Beispiele veranschaulicht. Die Erfindung ist jedoch nicht auf die Beispiele limitiert.

**Anwendungsbeispiele**

In den nachfolgend aufgeführten Anwendungsbeispielen wurden jeweils Mischungen von folgenden Carboxamiden der allgemeinen Formel (I) (Gruppe 1) mit den jeweils angegebenen Mischungspartnern (Strukturformeln siehe oben) getestet.

Eingesetzte Carboxamide der Formel (I):

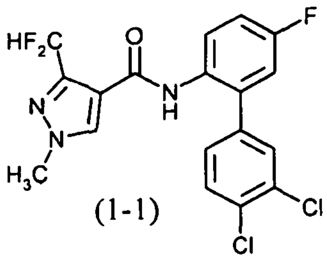
(1-1)

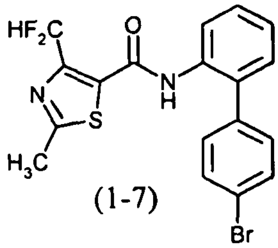
(1-7)

Beispiel A (Vergleichsbeispiel)

**Pyrenophora teres-Test (Gerste) / kurativ**

Lösungsmittel : 50 Gewichtsteile N,N-Dimethylacetamid

Emulgator : 1 Gewichtsteil Alkylarylpolyglykolether

Zur Herstellung einer zweckmäßigen Wirkstoffzubereitung vermischt man 1 Gewichtsteil Wirkstoff oder Wirkstoffkombination mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidiensuspension von *Pyrenophora teres* besprüht. Die Pflanzen verbleiben 48 Stunden bei 20°C und 100 % relativer Luftfeuchtigkeit in einer Inkubationskabine. Anschließend werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle A

| **Pyrenophora teres-Test (Gerste) / kurativ** | | | |
|---|---|---|---|
| Wirkstoffe | Aufwandmenge an Wirkstoff in g/ha | Wirkungsgrad in % | |
| | | gef.* | ber.** |
| **(1-1)** | 25 | 43 | |
| (2-2) Fluoxastrobin | 25 | 0 | |
| (3-17) Tebuconazole | 25 | 29 | |

(fortgesetzt)

| Pyrenophora teres-Test (Gerste) / kurativ | | | |
|---|---|---|---|
| Wirkstoffe | Aufwandmenge an Wirkstoff in g/ha | Wirkungsgrad in % | |
| | | gef.* | ber.** |
| **(1-1)** + (2-2) Fluoxastrobin (1:1) | 25 + 25 | 71 | 43 |
| **(1-1)** + (3-17) Tebuconazole (1:1) (erfindungsgemäß) | 25 + 25 | 71 | 60 |
| * gef. = gefundene Wirkung<br>** ber. = nach der Colby-Formel berechnete Wirkung | | | |

Beispiel B (Vergleichsbeispiel)

**Erysiphe-Test (Gerste) / protektiv**

Lösungsmittel : 50 Gewichtsteile N,N-Dimethylacetamid

Emulgator : 1 Gewichtsteil Alkylarylpolyglykolether

Zur Herstellung einer zweckmäßigen Wirkstoffzubereitung vermischt man 1 Gewichtsteil Wirkstoff oder Wirkstoffkombination mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelags werden die Pflanzen mit Sporen von *Erysiphe graminis f.sp. hordei* bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle B

| Erysiphe-Test (Gerste) / protektiv | | | |
|---|---|---|---|
| Wirkstoffe | Aufwandmenge an Wirkstoff in g/ha | Wirkungsgrad in % | |
| | | gef.* | ber.** |
| **(1-1)** | 12,5 | 0 | |
| (2-4) Trifloxystrobin | 12,5 | 78 | |
| (3-15) Prothioconazole | 12,5 | 67 | |
| **(1-1)** + (2-4) Trifloxystrobin (1:1) | 12,5 + 12,5 | 94 | 78 |
| **(1-1)** + (3-15) Prothioconazole (1:1) (erfindungsgemäß) | 12,5 + 12,5 | 89 | 67 |
| * gef. = gefundene Wirkung<br>** ber. = nach der Colby-Formel berechnete Wirkung | | | |

Beispiel C (Vergleichsbeispiel)

**Puccinia-Test (Weizen) / kurativ**

Lösungsmittel: 50 Gewichtsteile N,N-Dimethylacetamid

Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Kondiensuspension von *Puccinia recondita* besprüht. Die Pflanzen verbleiben 48 Stunden bei 20°C und 100 % relativer Luftfeuchte in einer Inkubationskabine.

Anschließend werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle C

| **Puccinia-Test (Weizen) / kurativ** | | | |
|---|---|---|---|
| Wirkstoffe | Aufwandmenge an Wirkstoff in g/ha | Wirkungsgrad in % | |
| | | gef.* | ber.** |
| **(1-1)** | 62,5 | 22 | |
| (19-10) Spiroxamine | 62,5 | 0 | |
| (6-14) | 62,5 | 44 | |
| (6-11) | 62,5 | 0 | |
| (2-11) Picoxystrobin | 62,5 | 78 | |
| **(1-1)** + (19-10) Spiroxamine (1:1) | 62,5 + 62,5 | 100 | 22 |
| **(1-1)** + (6-14) (1:1) | 62,5 + 62,5 | 67 | 57 |
| **(1-1)** + (6-11) (1:1) | 62,5 + 62,5 | 44 | 22 |
| **(1-1)** + (2-11) Picoxystrobin (1:1) | 62,5 + 62,5 | 89 | 83 |
| * gef. = gefundene Wirkung<br>** ber. = nach der Colby-Formel berechnete Wirkung | | | |

<u>Beispiel D</u> (Vergleichbeispiel)

**Gibberella zeae-Test (Gerste) / kurativ**

Lösungsmittel: 50 Gewichtsteile N,N-Dimethylacetamid

Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidien-Suspension von Gibberella zeae besprüht. Die Pflanzen verbleiben 24 Stunden bei 22°C und 100 % relativer Luftfeuchtigkeit in einer Inkubationskabine. Anschließend besprüht man die Pflanzen mit der Wirkstoffzubereitung in der angegebenen Aufwandmenge. Nach Antrocknen des Spritzbelages verbleiben die Pflanzen in einem Gewächshaus unter lichtdurchlässigen Inkubationshauben bei einer Temperatur von ca. 22°C und einer relativen Luftfeuchtigkeit von ca. 100 %.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle D

| **Gibberella zeae-Test (Gerste) / kurativ** | | | |
|---|---|---|---|
| Wirkstoffe | Aufwandmenge an Wirkstoff in g/ha | Wirkungsgrad in % | |
| | | gef.* | ber.** |
| **(1-1)** | 62,5 | 40 | |
| (2-12) Pyraclostrobin | 62,5 | 80 | |
| (3-12) Epoxyconazole | 62,5 | 0 | |
| **(1-1)** + (2-12) Pyraclostrobin (1:1) | 62,5 + 62,5 | 90 | 88 |
| **(1-1)** + (3-12) Epoxyconazole (1:1) (erfindungsgemäβ) | 62,5 + 62,5 | 60 | 40 |
| * gef. = gefundene Wirkung<br>** ber. = nach der Colby-Formel berechnete Wirkung | | | |

<u>Beispiel E</u> (Vergleichbeispiel)

**Sphaerotheca fuliginea -Test (Gurke) / protektiv**

Lösungsmittel: 24,5 Gewichtsteile Aceton
24,5 Gewichtsteile Dimethylacetamid

Emulgator: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Sphaerotheca fuliginea* inokuliert.

Die Pflanzen werden dann bei ca. 23°C und einer relativen Luftfeuchtigkeit von ca. 70% im Gewächshaus aufgestellt.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle E

| **Sphaerotheca fuliginea-Test (Gurke) / protektiv** | | | |
|---|---|---|---|
| Wirkstoffe | Aufwandmenge an Wirkstoff in g/ha | Wirkungs grad in % | |
| | | gef.* | ber.** |
| **(1-1)** | 4<br>2<br>1<br>0,5 | 30<br>36<br>16<br>0 | |
| **(1-7)** | 2<br>1<br>0,5 | 0<br>0<br>0 | |

(fortgesetzt)

| Sphaerotheca fuliginea-Test (Gurke) / protektiv | | | |
|---|---|---|---|
| Wirkstoffe | Aufwandmenge an Wirkstoff in g/ha | Wirkungs grad in % | |
| | | gef.* | ber.** |
| (2-1) Azoxystrobin | 0,5 | 20 | |
| (2-2) Fluoxastrobin | 1 | 0 | |
| (2-4) Trifloxystrobin | 2 | 10 | |
| (2-12) Pyraclostrobin | 2 | 0 | |
| (3-15) Prothioconazole | 1 | 43 | |
| (3-17) Tebuconazole | 1 | 10 | |
| (3-21) Bitertanol | 1 | 0 | |
| (4-2) Tolylfluanid | 20 | 0 | |
| (6-6) Fenhexamid | 20 | 0 | |
| (6-14) Penthiopyrad | 4 | 0 | |
| (7-1) Mancozeb | 20 | 0 | |
| (7-4) Propineb | 20 | 11 | |
| (9-3) Pyrimethanil | 20 | 0 | |
| (12-4) Iprodione | 20 | 0 | |
| (19-2) Chlorothalonil | 20 | 0 | |
| (19-10) Spiroxamine | 20 | 0 | |
| (22-1) | 2 | 11 | |
| (22-2) | 1 | 22 | |
| **(1-1)** + (2-1) Azoxystrobin (1:1) | 0,5 + 0,5 | 87 | 20 |
| **(1-7)** + (2-1) Azoxystrobin (1:1) | 0,5 + 0,5 | 63 | 20 |
| **(1-1)** + (2-2) Fluoxastrobin (1:1) | 1 + 1 | 95 | 16 |
| **(1-7)** + (2-2) Fluoxastrobin (1:1) | 1 + 1 | 92 | 0 |
| **(1-1)** + (2-4) Trifloxystrobin (1:1) | 2 + 2 | 57 | 42 |
| **(1-7)** + (2-4) Trifloxystrobin (1:1) | 2 + 2 | 93 | 10 |
| **(1-1)** + (2-12) Pyraclostrobin (1:1) | 2 + 2 | 53 | 36 |
| **(1-1)** + (3-15) Prothioconazole (1:1) (erfindungsgemäß) | 1 + 1 | 70 | 52 |
| **(1-1)** + (3-17) Tebuconazole (1:1) (erfindungsgemäß) | 1 + 1 | 90 | 24 |
| **(1-1)** + (3-21) Bitertanol (1:1) (erfindungsgemäß) | 1 + 1 | 50 | 16 |
| **(1-1)** + (4-2) Tolylfluanid (1:10) | 2 + 20 | 98 | 36 |
| **(1.1)** + (6-6) Fenhexamid (1:10) | 2 + 20 | 85 | 36 |
| **(1-1)** + (6-14) Penthiopyrad (1:1) | 4 + 4 | 82 | 30 |
| **(1-1)** + (7-1) Mancozeb (1:10) | 2 + 20 | 93 | 36 |
| **(1-1)** + (7-4) Propineb (1:10) | 2 + 20 | 65 | 43 |
| **(1-1)** + (9-3) Pyrimethanil (1:10) | 2 + 20 | 96 | 36 |
| **(1-1)** + (12-4) Iprodione (1:10) | 2 + 20 | 74 | 36 |
| **(1-1)** + (19-2) Chlorothalonil (1:10) | 2 + 20 | 91 | 36 |

(fortgesetzt)

| Sphaerotheca fuliginea-Test (Gurke) / protektiv | | | |
|---|---|---|---|
| Wirkstoffe | Aufwandmenge an Wirkstoff in g/ha | Wirkungs grad in % | |
| | | gef.* | ber.** |
| **(1-1)** + (19-10) Spiroxamine (1:10) | 2 + 20 | 100 | 36 |
| **(1-1)** + (22-1) (1:1) | 2 + 2 | 67 | 43 |
| **(1-1)** + (22-2) (1:1) | 1 + 1 | 94 | 34 |
| * gef. = gefundene Wirkung<br>** ber. = nach der Colby-Formel berechnete Wirkung | | | |

<u>Beispiel F</u> (Vergleichbeispiel)

**Alternaria solani - Test (Tomate) / protektiv**

Lösungsmittel: 24,5 Gewichtsteile Aceton
24,5 Gewichtsteile Dimethylacetamid

Emulgator: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Alternaria solani* inokuliert.

Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle F

| Alternaria solani - Test (Tomate) / protektiv | | | |
|---|---|---|---|
| Wirkstoffe | Aufwandmenge an Wirkstoff in g/ha | Wirkungsgrad in % | |
| | | gef.* | ber.** |
| **(1-1)** | 1<br>0,5 | 61<br>42 | |
| **(1-7)** | 1<br>0,5 | 63<br>28 | |
| (2-3) | 0,5 | 22 | |
| (3-3) Propiconazole | 0,5 | 3 | |
| (5-3) Benthiavalicarb | 1 | 5 | |
| (8-4) Metalaxyl-M | 0,5 | 7 | |
| (8-5) Benalaxyl-M | 0,5 | 14 | |
| **(1-7)** + (2-3) (1:1) | 0,5 + 0,5 | 67 | 44 |
| **(1-7)** + (3-3) Propiconazole (1:1) | 0,5 + 0,5 | 56 | 30 |

(fortgesetzt)

| Alternaria solani - Test (Tomate) / protektiv | | | |
|---|---|---|---|
| Wirkstoffe | Aufwandmenge an Wirkstoff in g/ha | Wirkungsgrad in % | |
| | | gef.* | ber.** |
| **(1-1)** + (5-3) Benthiavalicarb (1:1) | 1 + 1 | 77 | 63 |
| **(1-1)** + (8-4) Metalaxyl-M (1:1) | 0,5 + 0,5 | 62 | 46 |
| **(1-1)** + (8-5) enafaxyl-M (1:1) | 0,5 + 0,5 | 67 | 50 |
| * gef. = gefundene Wirkung<br>** ber. = nach der Colby-Formel berechnete Wirkung | | | |

Beispiel G (Vergleichsbeispiel)

**Phytophthora infestans - Test (Tomate) / protektiv**

Lösungsmittel: 24,5 Gewichtsteile Aceton
24,5 Gewichtsteile Dimethylacetamid

Emulgator: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Phytophthora infestans* inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle G

| Phytophthora infestans - Test (Tomate) / protektiv | | | |
|---|---|---|---|
| Wirkstoffe | Aufwandmenge an Wirkstoff in g/ha | Wirkungsgrad in % | |
| | | gef.* | ber.** |
| **(1-1)** | 10<br>5<br>1<br>0,5 | 0<br>0<br>0<br>0 | |
| (4-2) Tolylfluanid | 10 | 0 | |
| (5-1) Iprovalicarb | 10<br>5 | 64<br>61 | |
| (5-3) Benthiavalicarb | 0,5 | 56 | |
| (19-13) Fenamidone | 0,5 | 41 | |
| **(1-1)** + (4-2) Tolylfluanid (1:10) | 1 + 10 | 51 | 0 |
| **(1-1)** + (5-1) Iprovalicarb (1:1) | 10 + 10<br>5 + 5 | 88<br>77 | 64<br>61 |

(fortgesetzt)

| Phytophthora infestans - Test (Tomate) / protektiv | | | |
|---|---|---|---|
| Wirkstoffe | Aufwandmenge an Wirkstoff in g/ha | Wirkungsgrad in % | |
| | | gef.* | ber.** |
| **(1-1)** + (5-3) Benthiavalicarb (1:1) | 0,5 + 0,5 | 73 | 56 |
| **(1-1)** + (19-13) Fenamidone (1:1) | 0,5 + 0,5 | 51 | 41 |
| * gef. = gefundene Wirkung<br>** ber. = nach der Colby-Formel berechnete Wirkung | | | |

Beispiel H (Verpleichsbeispiel)

**Botrytis cinerea - Test (Bohne) / protektiv**

Lösungsmittel: 24,5 Gewichtsteile Aceton
24,5 Gewichtsteile Dimethylacetamid

Emulgator: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle H

| Botrytis cinerea - Test (Bohne) / protektiv | | | |
|---|---|---|---|
| Wirkstoffe | Aufwandmenge an Wirkstoff in g/ha | Wirkungsgrad in % | |
| | | gef.* | ber.** |
| **(1-1)** | 5 | 54 | |
| (9-3) Pyrimethanil | 5 | 4 | |
| (12-4) Iprodione | 5 | 13 | |
| **(1-1)** + (9-3) Pyrimethanil (1:1) | 5+5 | 92 | 56 |
| **(1-1)** + (12-4) Iprodione (1:1) | 5+5 | 100 | 60 |
| * gef. = gefundene Wirkung<br>** ber. = nach der Colby-Formel berechnete Wirkung | | | |

Beispiel I (Vergleichsbeispiel)

**Alternaria mali -Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Sporensuspension von *Alternaria mali* verwendet. Nach 5 Tagen Inkubation bei Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle I

| **Alternaria mali -Test (in vitro) / Mikrotiterplatten** | | | |
|---|---|---|---|
| Wirkstoffe | Aufwandmenge an Wirkstoff in ppm | Wirkungsgrad in % | |
| | | gef.* | ber.** |
| **(1-1)** | 0,03<br>0,003 | 51<br>25 | |
| (10-3) Carbendazim | 0,03 | 15 | |
| (19-3) Fenamidone | 0,003 | 2 | |
| (20-1) Pencycuron | 0,003 | 11 | |
| **(1-1)** + (10-3) Carbendazim (1:1) | 0,03+0,03 | 79 | 59 |
| **(1-1)** + (19-3) Fenamidone (1:) | 0,003+0,003 | 35 | 27 |
| **(1-1)** + (20-1) Pencycuron (1:1) | 0,003+0,003 | 67 | 33 |
| * gef. = gefundene Wirkung<br>** ber. = nach der Colby-Formel berechnete Wirkung | | | |

Beispiel J Vergleichsbeispiel)

**Rhizoctonia solani-Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Myzelsuspension von *Rhizoctonia solani* verwendet. Nach 5 Tagen Inkubation bei Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle J

| **Rhizoctonia solani-Test (in vitro) / Mikrotiterplatten** | | | |
|---|---|---|---|
| Wirkstoffe | Aufwandmenge an Wirkstoff in ppm | Wirkungsgrad in % | |
| | | gef.* | ber.** |
| **(1-1)** | 0,3<br>0,1 | 80<br>40 | |
| (17-1) Fosetyl-Al | 0,3 | 24 | |
| (11-2) Propamocarb | 0,1 | 25 | |
| **(1-1)** + (17-1) Fosetyl-Al (1:1) | 0,3+0,3 | 98 | 85 |

(fortgesetzt)

| Rhizoctonia solani-Test (in vitro) / Mikrotiterplatten | | | |
|---|---|---|---|
| Wirkstoffe | Aufwandmenge an Wirkstoff in ppm | Wirkungsgrad in % | |
| | | gef.* | ber.** |
| **(1-1)** + (11-2) Propamocarb (1:1) | 0,1+0,1 | 88 | 55 |
| * gef. = gefundene Wirkung<br>** ber. = nach der Colby-Formel berechnete Wirkung | | | |

Beispiel K (Vergleichsbeispiel)

**Septoria tritici-Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Sporensuspension von *Septoria tritici* verwendet. Nach 7 Tagen Inkubation bei Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle K

| Septoria tritici-Test (in vitro) / Mikrotiterplatten | | | |
|---|---|---|---|
| Wirkstoffe | Aufwandmenge an Wirkstoff in ppm | Wirkungsgrad in % | |
| | | gef.* | ber.** |
| **(1-1)** | 0,01 | 15 | |
| (14-3) Triazoxide | 0,01 | 29 | |
| **(1-1)** + (14-3) Triazoxide (1:1) | 0,01+0,01 | 69 | 40 |
| * gef. = gefundene Wirkung<br>** ber. = nach der Colby-Formel berechnete Wirkung | | | |

Beispiel L (Vergleichsbeispiel)

**Sphaerotheca fuliginea - Test (Gherkin) / protektiv**

Zur Herstellung einer zweckmäßigen Wirkstoffzubereitung wird die zu testende Substanz in einer Mischung aus Aceton/Tween/Wasser homogenisiert. Die Suspension wird mit Wasser auf die gewünschte Konzentration verdünnt.

Gherkin Pflanzen (Vert petit de Paris Varietät) werden in Startercups auf 50/50 Torfboden-Puzzolanerde-Substrat ausgesät und bei 20°C/23°C angezogen. Im 2-Blatt-Stadium werden die Pflanzen mit der Wirkstoffzubereitung in der angegebenen Aufwandmenge besprüht.

Zur Prüfung auf protektive Wirksamkeit werden die Pflanzen nach 24 h mit einer wässrigen Sporensuspension von *Sphaerotheca fuliginea* (100000 Sporen/ml) besprüht. Die Pflanzen verbleiben anschließen bei 20°C/25°C und 60/70 % relativer Luftfeuchtigkeit.

21 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der Wirkstoffkombination größer ist als die berechnete, d.h. dass ein synergistischer Effekt vorliegt.

Tabelle L

| Sphaerotheca fuliginea - Test (Gherkin) / protectiv | | | |
|---|---|---|---|
| Wirkstoffe | Aufwandmenge an Wirkstoff in ppm | Wirkungsgrad in % | |
| | | gef.* | ber.** |
| **(1-1)** | 8 | 60 | |
| (6-2) Boscalid | 8 | 50 | |
| **(1-1)**+(6-2) Boscalid (1:1) | 8+8 | 98 | 80 |
| * gef. = gefundene Wirkung<br>** ber. = nach der Colby-Formel berechnete Wirkung | | | |

## Patentansprüche

1. Synergistische fungizide Wirkstoffkombinationen enthaltend das Carboxamid (1-1) *N*-(3',4'-Dichlor-5-fluor-1,1'-biphenyl-2-yl)-3-(difluormethyl)-1-methyl-1*H*-pyrazol-4-carboxamid (Gruppe 1) und mindestens Triazol, ausgewählt aus der Gruppe bestehend aus

    (3-3) Propiconazole,
    (3-6) Cyproconazole,
    (3-12) Epoxiconazole,
    (3-15) Prothioconazole,
    (3-17) Tebuconazole,
    (3-19) Metconazole und
    (3-21) Bitertanol.

2. Verwendung von Wirkstoffkombinationen gemäß Anspruch 1 zum Bekämpfung von unerwünschten phytopathogenen Pilzen.

3. Verfahren zum Bekämpfen von unerwünschten phytopathogenen Pilzen, **dadurch gekennzeichnet, dass** man Wirkstoffkombinationen gemäß Anspruch 1 auf die unerwünschten phytopathogenen Pilze und/oder deren Lebensraum ausbringt.

4. Verfahren zum Herstellen von fungiziden Mitteln, **dadurch gekennzeichnet, dass** man Wirkstoffkombinationen gemäß Anspruch 1 mit Streckmitteln und/ oder oberflächenaktiven Stoffen vermischt.

## Claims

1. Synergistic fungicidal active compound combinations comprising the carboxamide (1-1) *N*-(3',4'-dichloro-5-fluoro-1,1'-biphenyl-2-yl)-3-(difloaromethyl)-1-methyl-1*H*-pyrazolo-4-carboxamide (group 1) and at least one triazole selected from the group consisting of

    (3-3) propiconazole,
    (3-6) cyproconazole,
    (3-12) epoxiconazole,
    (3-15) prothioconazole,
    (3-17) tebuconazole,
    (3-19) metconazole and
    (3-21) bitertanol.

2. Use of active compound combinations according to Claim 1 for controlling unwanted phytopathogenic fungi.

3. Method for controlling unwanted phytopathogenic fungi, **characterized in that** active compound combinations according to Claim 1 are applied to the unwanted phytopathogenie fungi and/or their habitat.

**4.** Process for preparing fungicide compositions, **characterized in that** active compound combinations according to Claim 1 are mixed with extenders and/or surfactants.

## Revendications

**1.** Associations fongicides synergiques de substances actives, contenant le carboxamide (1-1) *N*-(3',4'-dichloro-5-fluoro-1,1'-biphényl-2-yl)-3-(difluorométhyl)-1-méthyl-1*H*-pyrazole-4-carboxamide (groupe 1) et au moins triazole choisi dans le groupe composé de

(3-3) propiconazole,
(3-6) cyproconazole,
(3-12) époxiconazole,
(3-15) prothioconazole,
(3-17) tébuconazole,
(3-19) metconazole et
(3-21) bitertanol.

**2.** Utilisation d'associations de substances actives selon la revendication 1, pour la lutte contre des champignons phytopathogènes indésirables.

**3.** Procédé pour la lutte contre des champignons phytopathogènes indésirables, **caractérisé en ce qu'**on applique des associations de substances actives selon la revendication 1 sur les champignons phytopathogènes indésirables et/ou leur habitat.

**4.** Procédé pour la préparation de compositions fongicides, **caractérisé en ce qu'**on mélange des associations de substances actives selon la revendication 1 avec des excipients et/ou des substances tensioactives.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10215292 A
- WO 0208197 A
- WO 0014071 A
- EP 0040345 A
- DE 2201063 A
- DE 2324010 A
- EP 0382375 A
- EP 0515901 A
- WO 9706171 A
- DE 19646407 A1
- EP 712396 B
- WO 9931979 A
- WO 03070705 A1
- WO 03070705 A
- DE 2551560 A
- DE 3406993 A
- EP 0196038 A
- WO 9616048 A
- EP 0329397 A


### In der Beschreibung aufgeführte Nicht-Patentliteratur


- Pesticide Manual. 1991, 249, 827
- **S.R. COLBY.** Calculating Synergistic and Antagonistic Responses of Herbicide Combinations. *Weeds,* 1967, vol. 15, 20-22